# EUROPÄISCHE PATENTANMELDUNG

(11) **EP 4 574 115 A1**
(43) Veröffentlichungstag der Anmeldung: **25.06.2025**
(21) Anmeldenummer: 24222378.2
(22) Anmeldetag: 20.12.2024
(51) Int. Cl.: A61F 13/06, A61F 13/08

(54) **MEDIZINISCHES BEKLEIDUNGSSTÜCK**

(30) Priorität: 22.12.2023 DE 102023136621
(71) Anmelder: KOB GmbH, 67752 Wolfstein (DE)
(72) Erfinder: TAMOUÉ, Ferdinand, 67752 Wolfstein (DE)
(74) Vertreter: Mathys & Squire

(57) **Zusammenfassung**

Die Erfindung betrifft ein medizinisches Bekleidungsstück zum Ausüben eines Kompressionsdrucks auf eine untere Extremität. Das medizinische Bekleidungsstück umfasst einen einteiligen Zuschnitt eines in Maschinenrichtung elastischen Textils mit einem ersten und einem zweiten Abschnitt in Maschinenquerrichtung. Der erste Abschnitt ist mit einer ersten Elastizität zum Anlegen an das Bein vorgesehen und der zweite Abschnitt ist mit einer zweiten Elastizität zum Anlegen an einen Fuß vorgesehen. Das medizinische Bekleidungsstück umfasst ferner Befestigungsmittel, die dazu angepasst sind, durch überlappende Verbindung von entgegengesetzten Enden des Zuschnitts in Maschinenrichtung das Bekleidungsstück mittels der Verschlusselemente die untere Extremität umschließend lösbar zu verschließen. Das Bekleidungsstück ist dazu angepasst ist, einen ersten Kompressionsdruck auf das Bein und einen zweiten Kompressionsdruck auf den Fuß auszuüben, wobei der erste Kompressionsdruck höher als der zweite Kompressionsdruck ist.

## Beschreibung

Die Erfindung betrifft ein medizinisches Bekleidungsstück.

Venenerkrankungen sind ein ernstes Problem, wobei die akute Form der tiefen Venenthrombose (TVT) und die Lungenembolie eine der Haupttodesursachen darstellen und die chronische Form eine der Hauptursachen für Arbeitsunfähigkeit ist.

Venenleiden sind auch deshalb ein so großes Problem, weil bis zu 50 % der erwachsenen Bevölkerung in der einen oder anderen Form davon betroffen sind, siehe hierzu Widmer LK. "Periphere Venenleiden. Prävalenz und sozialmedizinische Bedeutung: Beobachtungen bei 4529 scheinbar gesunden Personen"; Basler Studie III. Bern: Hans Huber, 1978.

Der physiologische Mechanismus des Venengeflechts der Fußsohle und des Malleolus gastrocnemius hat einen starken Einfluss auf den Blutrückfluss und gewährleistet die Gesundheit der Venen. Die Plantarvenen spielen eine unverzichtbare Rolle im venösen System der unteren Gliedmaßen. Das vom Herzen zu den distalen Extremitäten des Körpers gepumpte Blut muss anschließend trotz vieler Hindernisse aufsteigen, wie z. B.: die Entfernung, die dem anfänglichen Druckeffekt auf Herzhöhe entgegenwirkt, und die Schwerkraft, die dazu neigt, eine Stagnation in den am weitesten vom Herzen entfernten Gefäßen zu begünstigen.

Um dieses Hindernis beim venösen Rückfluss zu überwinden, begünstigen die Muskelkontraktionen der unteren Gliedmaßen, die Venenklappen und die Lymphklappen (Lymphangion) den erwarteten Flüssigkeitsstrom im menschlichen Körper. Während des Blutflusses kommt es zu einer Kompression der Plantarvenen, die sich bei jedem Schritt entleeren. Darüber hinaus gibt es ein ganzes Kommunikationssystem zwischen den Plantarvenen und den dorsalen Venen, das den venösen Rückfluss aus dem tief liegenden Geflecht erleichtert.

Der venöse Rückfluss ist der retrograde Rückstrom des Blutes zum Herzen. In Ruhe muss der venöse Rückfluss gleich dem Herzzeitvolumen sein, da das Herz-Kreislauf-System im Wesentlichen ein geschlossener Kreislauf ist. Wenn der venöse Rückfluss niedriger ist als dieser, ist Kompressionsbekleidung, wie sie in dieser Erfindung vorgestellt wird, erforderlich.

Zur Kompressionsbekleidung gehören Kompressionsstrümpfe, Kompressionsbandagen und intermittierende Kompressionsvorrichtungen. Kompressionsartikel sind teilweise sehr gut an die Anatomie des Patienten angepasst und können eine patientenspezifische Kompressionsdruckkurve aufweisen. Das macht diese Produkte sicher in der Anwendung und gewährleistet die therapeutische Wirksamkeit. Schwerere Ödeme, venöse und lymphatische Insuffizienz werden z. B. durch Umwickeln der betroffenen Extremitäten mit Bandagen zur Kompressionstherapie behandelt.

Bandagieren wird meist von medizinischen Fachkräften und manchmal auch von unterwiesenen Laien durchgeführt. Das Anlegen von Verbänden erfordert eine gewisse Geschicklichkeit und Erfahrung. Der Verband wird in der Regel in mehreren Schichten (Binden) angelegt, wobei die verschiedenen Binden unterschiedliche Funktionen erfüllen. Bei mehrschichtigen Verbänden dient die erste Schicht in der Regel als Hautschutz und Polster (Druckverteilungsschicht), und eine zweite Schicht ist eine Kompressionsschicht, die das Elastizitätsmodul der ersten Bandage erhöht.

Einerseits ist die Kompressionstherapie nur dann wirksam, wenn die Binden fest genug angelegt werden. Andererseits muss darauf geachtet werden, dass die Bandagierung nicht zu einer Einschnürung der Gliedmaßen führt und ein erholsamer Schlaf noch möglich ist.

Die Verwendung von Kompressionskleidung ist begrenzt, da offene Wunden und knöcherne Vorsprünge an den Gliedmaßen sehr empfindlich auf Druckspitzen reagieren. Daher könnte die Entwicklung eines viskoelastischen adaptiven Kompressionskleidungsstücks für die Kompressionstherapie nützlich sein.

Die US 6,152,893 offenbart eine Kompressionsvorrichtung zum Anlegen einer vorbestimmten Kompression an Gliedmaße, die eine biegsame, nicht dehnbare Folie umfasst, die um Gliedmaße gewickelt wird.

Aus der WO 97/46181 ist ein therapeutisches Kompressionskleidungsstück mit einer Vielzahl von Paaren von Bandagen bekannt, die den Körper oder die Gliedmaßen umschließen, einstückig mit einem zentralen Umhüllungsbereich verbunden sind und sich in entgegengesetzter Richtung von beiden Seiten des zentralen Bereichs nach außen erstrecken, um das Körperteil zu umschließen.

Die EP 3 210 581 B1 beschreibt Wickel zur Kompressionsbehandlung von Lymphödemen am Fuß, mit einem Sohlenteil und Seitenteilen. An den Sohlenabschnitt schließen sich seitlich Seitenabschnitte an, die in einem Abschnitt miteinander verbunden sind, der beim Anlegen der Fußbandage oberhalb der Ferse zu liegen kommt. Die Fußbandage besteht ferner aus mindestens einem distalen Gurtabschnitt aus einem elastischen Material, der um die Ferse gewickelt werden kann und an einem Seitenabschnitt befestigbar ist. Er ermöglicht die Ausübung von Druck auf den Fuß, wobei jeder distale Gurtabschnitt mindestens einen Zuggurt aufweist, an dem Befestigungsmöglichkeiten, z.B. Schlaufen, angeordnet sind.

Die EP 2 854 721 B1 offenbart ein Kompressionsbekleidungsstück mit einem Körper und einer Vielzahl von Bändern, die sich von diesem erstrecken, wobei mindestens eines der Bänder ein zweischichtiges Messsystem aufweist. Die Kompressionsmessung erfolgt durch zwei Schichten, die eine untere, dehnbare Schicht mit Markierungen und eine obere, an einem Ende angebrachte Schicht umfassen, wobei die Markierungen auf der dehnbaren Basisschicht nur sichtbar sind, wenn das Messsystem gedehnt wird.

Aus der EP 3 512 478 B1 ist ein Ärmel eines Kompressionsbekleidungsstücks mit einem länglichen Arm, der an seinem ersten Ende eine Vielzahl von Befestigungsteilen aufweist, die mit dem Befestigungselement des zweiten Endes des Bekleidungsstücks verbindbar sind. Das zweite Ende des Bekleidungsstücks ist ein Teil des schlauchförmigen Teils des Bekleidungsstücks, in den die Gliedmaße während der Kompression eingeführt werden. Der Arm des Stretch-Bekleidungsstücks hat eine Innen- und eine Außenseite und kann während des Einwickelns der Gliedmaßen in Längs- und Querrichtung gedehnt werden.

Rabe E, Földi E, Gerlach H, Jünger M, Lulay G, Miller A, Protz K, Reich-Schupke S, Schwarz T, Stücker M, Valesky E, Pannier F, Leitlinie: Medizinische Kompressionstherapie der Extremitäten mit Medizinischem Kompressionsstrumpf (MKS), Phlebologischem Kompressionsverband (PKV) und Medizinischen adaptiven Kompressionssystemen (MAK). AWMF-Registernummer: 037/005 beschreibt weitere Kompressionsverbände.

Aus Dalen IIE, Alpert JS. Natürlicher Verlauf der Lungenembolie. Prog Cardiovasc Dis 1975; 17: 257-70 sind adaptive Kompressionssysteme, auch Kompressionswickel genannt, bekannt, die es Patienten ermöglichen, die Kompressionstherapie selbst handzuhaben. Viele Patienten scheitern jedoch daran, Strümpfe selbst anzuziehen, weil sie nicht verstellbar sind. Es hat sich ferner gezeigt, dass die oben beschriebenen Systeme immer noch einen grundlegenden Nachteil haben, nämlich die Komplexität der Anwendung. Die derzeitigen adaptiven Kompressionsvorrichtungen sind auch nicht darauf ausgelegt, einen Zielgrenzflächendruck (2-8 kPa) zu gewährleisten, wie er in medizinischen Leitlinien empfohlen wird. Schließlich müssen übliche Kompressionsstrümpfe sehr elastisch sein und eine hohe Dehnbarkeit aufweisen, damit man sie an- und ausziehen kann.

Es ist daher eine Aufgabe der vorliegenden Erfindung ein medizinisches Bekleidungsstück zu schaffen, das die Komplexität der Anwendung reduziert und dabei gleichzeitig einen Zielgrenzflächendruck von 2-8 kPa gewährleistet. Ferner ist es eine Aufgabe der Erfindung, eine adaptive Kompressionsvorrichtung wie eine Bandage oder einen Strumpf vorzusehen, die leicht anzuziehen ist.

Diese Aufgabe wird durch den Gegenstand der unabhängigen Ansprüche gelöst. Vorteilhafte Ausgestaltungen und Weiterbildungen der Erfindung sind in den Unteransprüchen beschrieben.

Erfindungsgemäß ist ein medizinisches Bekleidungsstück zum Ausüben eines Kompressionsdrucks auf eine untere Extremität vorgesehen. Das medizinische Bekleidungsstück umfasst einen einteiligen Zuschnitt eines in Maschinenrichtung elastischen Textils mit einem ersten und einem zweiten Abschnitt in Maschinenquerrichtung. Der erste Abschnitt ist mit einer ersten Elastizität zum Anlegen an das Bein vorgesehen und der zweite Abschnitt ist mit einer zweiten Elastizität zum Anlegen an einen Fuß vorgesehen. Das medizinische Bekleidungsstück umfasst ferner Befestigungsmittel, die dazu angepasst sind, durch überlappende Verbindung von entgegengesetzten Enden des Zuschnitts in Maschinenrichtung das Bekleidungsstück mittels der Verschlusselemente die untere Extremität umschließend lösbar zu verschließen. Das Bekleidungsstück ist dazu angepasst ist, einen ersten Kompressionsdruck auf das Bein und einen zweiten Kompressionsdruck auf den Fuß auszuüben, wobei der erste Kompressionsdruck höher als der zweite Kompressionsdruck ist.

Hierbei ist es zweckmäßig, wenn der Zuschnitt aus einem elastischen Textil gefertigt ist, das einen Verbund aus einer Basisschicht und einer Oberschicht aufweist, wobei die beiden Schichten im ungedehnten Zustand mittels Nähwirkverfahren über in Maschinenrichtung laufende und in Maschinenquerrichtung jeweils voneinander beabstandete elastische Nähfäden miteinander verbunden sind, und wobei der Verbund in Maschinenquerrichtung des Nähwirkverfahrens in mindestens zwei unterscheidbare Abschnitte unterteilt ist, wobei der erste Abschnitt und der zweite Abschnitt unterschiedliche Dehnbarkeiten in Maschinenrichtung aufweisen.

Dabei ist es von Vorteil, wenn der Zuschnitt einen dritten Abschnitt zum Anlegen an die Ferse aufweist, der eine dritte Elastizität aufweist.

Um das Anlegen des medizinischen Bekleidungsstücks zu erleichtern, ist es von Vorteil, wenn der erste und zweite Abschnitt erste bzw. zweite Befestigungsmittel aufweisen, so dass das elastische Textil um das Bein bzw. den Fuß gelegt und auf sich selbst befestigt werden kann.

Um das Anlegen des Bekleidungsstückes an Fuß und Bein zu erleichtern, ist es zweckmäßig, wenn der dritte Abschnitt keine Elastizität in Maschinenrichtung aufweist und dazu vorgesehen ist, einen Brückenabschnitt zwischen erstem und zweitem Abschnitt auszubilden.

Hierbei ist es zweckmäßig, wenn der zweite Abschnitt ein Elastizitätsmodul von ≤ 6 kPa, bei 40% Dehnung und bei einer spezifischen Spannung von 1 N/cm aufweist.

Weiter ist es zweckmäßig, wenn der erste Abschnitt für einen Therapiedruck von 2-8 kPa mit 40-45% ausgelegt ist.

Es ist ebenfalls von Vorteil, wenn der erste Abschnitt ein Elastizitätsmodul von ≤ 40 kPa bei einer maximalen Spannung von 8 N/cm aufweist.

Hierbei ist es zweckmäßig, wenn im ersten und zweiten Abschnitt jeweils nur ein Befestigungsmittel vorgesehen ist.

Für eine einfache Befestigung des medizinischen Bekleidungsstücks am Körper eines Benutzers ist es zweckmäßig, wenn das Befestigungsmittel Verschlusselemente mit klebender Beschichtung, Klettverschlüsse oder Reißverschlüsse umfasst.

Für eine genaue Einstellung des Kompressionsdrucks auf den Fuß und das Bein ist es zweckmäßig, wenn der der erste und/oder zweite Abschnitte Indikatoren oder Markierungen für die Verschlusselemente aufweisen, um einen definierten Kompressionsdruck bei Anlegen des Bekleidungsstücks auf Bein und Fuß auszuüben.

Für eine gute Anpassung des medizinischen Bekleidungsstücks an den Beinbereich ist es zweckmäßig, wenn der erste Abschnitt des Zuschnitts eine rechteckige Form aufweist.

Für eine gute Anpassung des medizinischen Bekleidungsstücks an den Fußbereich ist es zweckmäßig, wenn der zweite Abschnitt des Zuschnitts eine "American Football"-Form oder eine Zitronen-Form aufweist.

Um eine Zugkraft von dem ersten Abschnitt zu dem zweiten Abschnitt bei einem Anlegen des medizinischen Bekleidungsstücks breitflächig zu übertragen, ist es zweckmäßig, wenn der dritte Abschnitt des Zuschnitts als Brückenabschnitt zwischen erstem und zweitem Abschnitt sanduhrförmig ausgebildet ist.

Darüber hinaus ist erfindungsgemäß ein Verfahren zum Anlegen des erfindungsgemäßen medizinischen Bekleidungsstücks vorgesehen, mit den Schritten: Auflegen des Zuschnitts des medizinischen Bekleidungsstücks auf einen flachen Untergrund; Betreten und Fixieren des zweiten Abschnitts durch einen Benutzer, Verschließen des Bekleidungsstücks im Fußbereich durch den Benutzer mit einem definierten zweiten Kompressionsdruck; Greifen des proximalen Endes im ersten Abschnitt durch einen Benutzer, während er noch auf dem zweiten Abschnitt steht; Ziehen des ersten Abschnitts nach oben in Richtung des Rumpfes des Benutzers, wobei aufgrund der Einteiligkeit des Zuschnitts ein Kraftschluss zwischen erstem und zweitem Abschnitt zustande kommt, sodass sich der erste Abschnitt in Beinrichtung und Maschinenquerrichtung des Zuschnitts lokal definiert das Bein umschließt; Verschließen des Bekleidungsstücks im Beinbereich durch den Benutzer mit einem definierten ersten Kompressionsdruck.

Hierbei ist es zweckmäßig, wenn sich der dritte Abschnitt, der an der Achillessehne der Ferse, Calcaneus, geführt wird, sich bei der Anwendung des medizinischen Bekleidungsstücks nicht dehnt.

Weiter ist erfindungsgemäß eine Verwendung des medizinisches Bekleidungsstück gemäß der Erfindung bei phlebo-lymphatischer Insuffizienz vorgesehen.

Es ist ferner ein elastisches Textil für medizinische Zwecke vorgesehen, das einen Verbund aus einer Basisschicht und einer Oberschicht aufweist, wobei die beiden Schichten im ungedehnten Zustand mittels Nähwirkverfahren über in Maschinenrichtung laufende und in Maschinenquerrichtung jeweils voneinander beabstandete elastische Nähfäden miteinander verbunden sind. Der Verbund ist in Maschinenquerrichtung des Nähwirkverfahrens in mindestens zwei unterscheidbare Abschnitte unterteilt, wobei der erste Abschnitt und der zweite Abschnitt unterschiedliche Dehnbarkeiten aufweisen.

Für eine einfache und kostengünstige Herstellung ist es zweckmäßig, wenn die Basisschicht und/oder die Oberschicht ein Vliesmaterial umfassen.

Dabei ist es von Vorteil, wenn die Basisschicht eine Dicke im Bereich zwischen 0,6 und 1,2 mm und/oder die Oberschicht eine Dicke im Bereich zwischen 0,6 und 1,2 mm aufweist.

Hierbei ist es zweckmäßig, wenn die elastischen Nähfäden thermoplastische Elastomergarne und/oder Multifilamente umfassen, die über die Basisschicht gelegt und an der Oberschicht durch eine Elastomerschmelze befestigt sind oder die Basisschicht und die Oberschicht im ungedehnten Zustand mittels Nähwirkverfahren durch Nähte miteinander verbinden.

Um eine Vielzahl von unterschiedlichen Dehnungseigenschaften zu erreichen, ist es vorteilhaft, wenn die elastischen Nähfäden aus einer Gruppe von Baumwoll-Spinnkreppfäden, Baumwoll-Zwirnkreppfäden, texturierten Polyamidgarnen, texturierten Polyestergarnen, Gummifäden oder Polyurethan-Elastanfäden oder einer Kombination hieraus ausgewählt werden.

Für eine Anwendung des elastischen Textils bei einem medizinischen Bekleidungsstück, insbesondere für den Fuß und Beinbereich, ist es von Vorteil, wenn der erste Abschnitt eine Dehnbarkeit in Maschinenrichtung von 20 - 95 % mit einem Elastizitätsmodul von kleiner 40 kPa und/oder der zweite Abschnitt eine Dehnbarkeit in Maschinenrichtung von 100% - 180 % mit einem Elastizitätsmodul von kleiner 6 kPa aufweist.

Hierbei ist es zweckmäßig, wenn der erste Abschnitt eine Dehnbarkeit in Maschinenrichtung von 60 % +/- 10 % und/oder der zweite Abschnitt eine Dehnbarkeit in Maschinenrichtung von 140 % +/- 10 % aufweist.

Vorteilhafterweise sind dabei im ersten und im zweiten Abschnitt die elastischen Nähfäden mit jeweils unterschiedlichen Feinheiten und/oder mit in Maschinenquerrichtung jeweils unterschiedlichen Fadendichten aufgebracht.

Für eine Einstellung der Elastizität des elastischen Textils in Maschinenrichtung ist es vorteilhaft, wenn im ersten und/oder im zweiten Abschnitt die elastischen Nähfäden innerhalb eines jeweiligen Abschnitts mit unterschiedlichen Feinheiten und/oder mit in Maschinenquerrichtung unterschiedlichen Fadendichten aufgebracht sind.

Dabei ist es von Vorteil, wenn der Verbund zumindest zwei elastische Nähfäden, insbesondere drei elastische Nähfäden mit unterschiedlicher Feinheit aufweist.

Für eine Anwendung des elastischen Textils bei einem medizinischen Bekleidungsstück, insbesondere für den Fuß und Beinbereich, ist es von Vorteil, wenn die elastischen Nähfäden im ersten Abschnitt eine Feinheit im Bereich zwischen 100 dtex und 200 dtex und im zweiten Abschnitt eine Feinheit im Bereich zwischen 100 dtex und 400 dtex aufweisen.

Bei einer Einstellung der Elastizität durch die Fadendichte ist es zweckmäßig, wenn die elastischen Nähfäden im ersten Abschnitt mit einer Fadendichte im Bereich zwischen 100 und 300 Fäden pro 10 cm und im zweiten Abschnitt mit einer Fadendichte im Bereich zwischen 100 und 300 Fäden pro 10 cm aufgebracht sind.

Für eine Anwendung des elastischen Textils bei einem medizinischen Kompressionsbekleidungsstück ist es von Vorteil, wenn der Verbund in der Lage ist, einer Belastung in Maschinenrichtung von mindestens 2 kPa standzuhalten, ohne sich während der Belastung in der Breite in Maschinenquerrichtung zu verengen.

Hierbei ist es zweckmäßig, wenn der erste und zweite Abschnitt mit unterschiedlicher Dehnbarkeit in Maschinenquerrichtung für das Anlegen des Verbundes an unterschiedliche anatomische Bereiche von Gliedmaßen, insbesondre für Bein, Ferse und Fuß vorgesehen sind.

Für eine Anwendung des elastischen Textils bei einem medizinischen Kompressionsbekleidungsstück für den Fuß- und Beinbereich ist es zweckmäßig, wenn der erste Abschnitt für das Anlegen des Verbundes an einen Beinbereich mit einem therapeutischen Zielgrenzdruck von 2-8 kPa und der zweite Abschnitt für das Anlegen des Verbundes an einen Fußbereich mit einem therapeutischen Zielgrenzdruck von 2-3 kPa vorgesehen sind.

Hierbei ist es zweckmäßig, wenn der Verbund ein genähtes Vlies oder ein Gelege ist, bei dem thermoplastische Urethan-Multifilamentgarne mit einer Feinheit von 133, 156 und 320 dtex systematisch auf die Oberfläche des Grundgewebes gelegt und entweder durch eine Elastomerschmelze oder durch Nähte an der Oberfläche des Obergewebes befestigt werden, um mindestens zwei Abschnitte mit unterschiedlicher Dehnbarkeit zu bilden.

Für eine Anwendung des elastischen Textils bei einem medizinischen Kompressionsbekleidungsstück für den Fuß- und Beinbereich ist es zweckmäßig, wenn der Verbund aus einem elastischen Textil besteht, das in der Maschinenquerrichtung in zwei unterscheidbare Abschnitte unterteilt ist, von denen der erste durch eine Dehnbarkeit von 60 % und der zweite durch eine Dehnbarkeit von 140 % für den Beinabschnitt bzw. den Fußabschnitt gekennzeichnet ist, wenn er als Fuß- und Beinkleidungsstück hergestellt wird.

Schließlich ist ein Verfahren zur Herstellung des elastischen Textils vorgesehen, mit den Schritten: Bereitstellen einer Basisschicht und einer Oberschicht; Festlegen einer Maschinenrichtung; Festlegen von mindestens zwei Abschnitten, deren Abschnittsgrenzen parallel zur Maschinenrichtung verlaufen; Auflegen von elastischen Fäden auf die Basisschicht, wobei in den jeweiligen Abschnitten eine unterschiedliche Anzahl von elastischen Fäden aufgelegt werden, so dass sich die Fadendichte in den Abschnitten unterscheidet und/oder wobei in den jeweiligen Abschnitten Fäden mit unterschiedlicher Feinheit aufgelegt werden; Verbinden der Basisschicht und der Oberschicht durch Vernagelung oder durch Aufschmelzen, so dass das elastische Textil in seiner Maschinenquerrichtung Abschnitte mit unterschiedlicher Dehnbarkeit und/oder Elastizitätsmodul aufweist;

Die Erfindung wird im Folgenden beispielsweise anhand der Zeichnungen näher erläutert. Es zeigen:
Fig. 1 eine schematische Seitenansicht einer Kettenwirkmaschine zur Herstellung eines Textils gemäß einem Ausführungsbeispiel der Erfindung;
Fig. 2 eine schematische Draufsicht auf die Kettenwirkmaschine gemäß Fig. 1;
Fig. 3 eine schematische Ansicht eines viskoelastischen Textils mit harmonisierter Dehnung gemäß einem Ausführungsbeispiel der Erfindung;
Fig. 4 eine fotografische Ansicht eines viskoelastischen medizinischen Bekleidungsstücks mit harmonisierter Dehnung gemäß einem Ausführungsbeispiel der Erfindung;
Fig. 5 eine schematische Ansicht einer Vorderseite eines medizinischen Bekleidungsstücks gemäß einem Ausführungsbeispiel der Erfindung;
Fig. 6 eine schematische Ansicht einer Rückseite eines medizinischen Bekleidungsstücks gemäß einem Ausführungsbeispiel der Erfindung; und
Fig. 7 eine fotografische Ansicht einer Rückseite eines medizinischen Bekleidungsstücks mit eingeklapptem Befestigungselement im zweiten Abschnitt gemäß einem Ausführungsbeispiel der Erfindung; und
Fig. 8 eine fotografische Ansicht eines ersten und zweiten Verfahrensschrittes zum Anlegen eines medizinischen Bekleidungsstücks gemäß einem Ausführungsbeispiel der Erfindung.

Im Folgenden werden für gleiche und gleichwirkende Bauteile dieselben Bezugszeichen verwendet.

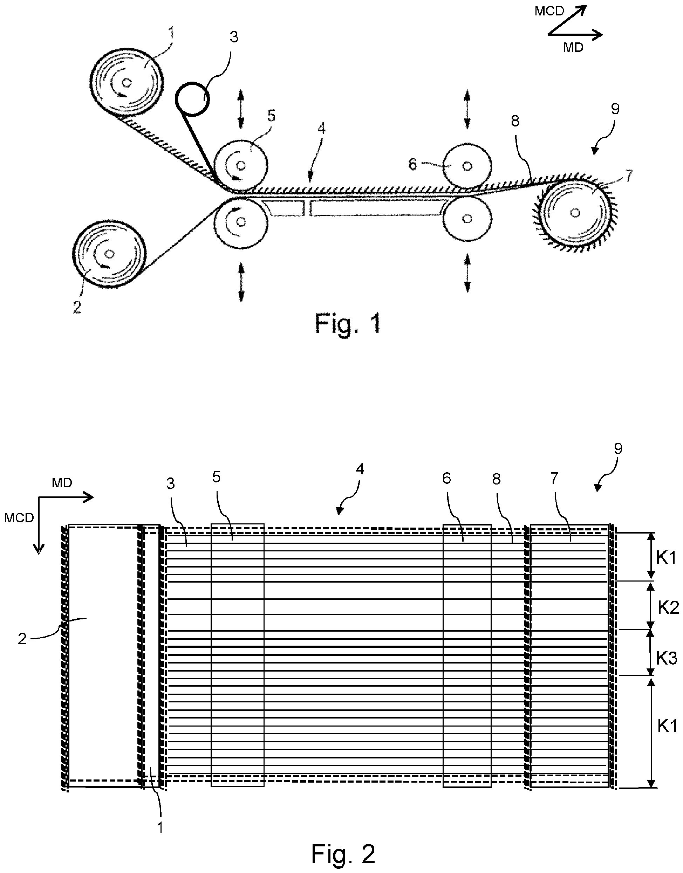

In Figur 1 ist dargestellt, wie zum einen eine Polsterschicht oder Basisschicht 1 aus einem Wattevlies und einer Stützschicht oder Oberschicht 2 hier als Thermobondvliesschicht aus einem Rollenmaterial zugeführt werden und in einem Nähwirkverfahren mittels einer Kettenwirkmaschine 4 verbunden werden. Dabei werden zuvor über eine Rollenführung 5 die beiden Lagen aufeinander fixiert. Das Nähwirkverfahren arbeitet dabei mit einem Haken, mittels dem die Schichten mit einem elastischen Nähfäden 3 übernäht werden. Der miteinander über den elastischen Nähfaden 3 verbundene Verbund 8 wird dann durch eine weitere Rollenführung 6 geführt und auf eine Rolle 7 aufgewickelt, um ein elastisches Textil 9 für medizinische Zwecke vorzusehen, das, wie noch beschrieben werden wird, als Textil für die Herstellung eines erfindungsgemäßen medizinischen Bekleidungsstücks 10 verwendet werden kann. Bei dem elastischen Nähfaden 3 handelt es sich um einen elastisch vorgedehnten Faden, der mit einer vorgegebenen Stichlänge und einer vorgegebenen Nähfadenspannung eingebracht wird, und so nach Entspannung des elastischen Materials zu einem Zusammenziehen des Verbundes 8 (Lagenverbundes oder Flächengebildes) führt.

Sofern die Begriffe Schicht oder Lage verwendet werden, bezeichnen diese den gleichen Gegenstand. Die beiden Schichten 1, 2 werden gemäß diesem Ausführungsbeispiel der Erfindung mittels eines Nähwirkverfahrens, hier vorzugsweise einem Malimo- bzw. MaliwattVerfahren verbunden, wobei die Schichten im ungedehnten Zustand mittels des elastischen Nähfadens 3 verbunden werden.

Der Vorteil bei einem Nähwirkverfahren besteht darin, dass gleichzeitig an mehreren Stellen eine Verbindung durchgeführt werden kann und die beiden Schichten 1, 2 nach der Verbindung nicht mehr voneinander getrennt werden können. Über die Auswahl der Stichlänge in Längsrichtung des Verbundes 8 (Flächengebildes), wobei unter Stichlänge der Abstand in Stichlängsrichtung zwischen zwei Stichen verstanden werden soll, und der Nähfadenspannung kann die Dehnbarkeit des elastischen Verbundes 8 eingestellt werden, so dass ein von Hand nicht mehr trennbarer und trotzdem kontrollierbar elastischer Verbund 8 aus den beiden Schichten 1, 2 besteht. Je nach Auswahl dieser Parameter zieht sich das fertige Flächengebilde des Verbundes 8 zusammen bei Entspannung und werden Falten im Material aufgeworfen.

Die Nähtechnik und die Stichlänge des Nähfadens werden dabei so reguliert, dass die Fasern auf der Basisschicht 1 oder Polsterschicht auf der einen Seite des Verbundes 8 aus den zwei Schichten 1, 2 Hautkomfort und Ausgleichsfunktionen besitzen und damit letztendlich ein medizinisches Bekleidungsstück 10 oder Kompressionsverband zwei erkennbar unterschiedliche Seiten aufweist, die für den Druckausgleich sehr funktional sind. Des Weiteren ist die Stichlänge dabei so einzustellen, dass die gewünschten absorbierenden sowie hautfreundlichen Eigenschaften der der Haut zugewandten Polsterschicht oder Basisschicht 1 erhalten bleiben.

Die Polsterschicht oder Basisschicht 1 ist dabei die einer Gliedmaße zugewandte Seite des medizinischen Bekleidungsstücks 10 oder Kompressionsverbandes und die Stützschicht oder Oberschicht 2 die hierauf aufgebrachte zweite Seite.

Das Malimo- bzw. Maliwattverfahren zum Verbinden der Schichten wird dabei wie im Stand der Technik bekannt eingesetzt. So kann z.B. die Elastizität durch Übernähen eines starren Vliessstoffes oder Textils mit dauerelastischen Elastanfäden in Längsrichtung unter Anwendung der Nähwirktechnik MALIWATT oder Malimo erhalten wurde. Die Nähwirktechnik MALIWATT bzw. Malimo ist in Malimo Nähwirktechnologie, Ploch, Böttcher, Scharch, VEB Fachbuchverlag Leipzig, 1978, 1. Auflage beschrieben.

Bevorzugt können beide Schichten 1, 2, also die Basisschicht 1 und/oder die Oberschicht 2 unelastisch ausgebildet sein und werden erst durch das Nähwirkverfahren elastifiziert. Besonders bevorzugt kann vorgesehen sein, dass mindestens eine der beiden Schichten 1, 2, jedoch bevorzugt beide der Schichten 1, 2 aus Polstermaterial und Stützschicht ein Vliesmaterial sind. Alternativ sind jedoch auch andere Materialien wie z.B. Gewebe, Gewirke, Gestricke oder Schäume einsetzbar. Dabei kann es sich bei der Basisschicht 1 um eine Wattevliesschicht, insbesondere eine Thermofusionsvliesschicht, handeln, die gegebenenfalls auch schon vorvernadelt sein kann. Dabei werden bei beiden Verfahren, nämlich dem Thermobonding als auch der der Thermofusion die Fasern der Vliese in einem Kämmereiverfahren in eine bestimmte Richtung gelegt und in einem Textilfunktionalisierungsverfahren als Vliesrollen vorbereitet und durch Temperatur oder durch Temperatur und Druck stabilisiert für die Weiterverarbeitung. Während des Thermofusionsverfahrens werden Fasern mit verschiedenen Schmelzpunkten durch Heißluft-Trockner miteinander verschmolzen. Im Thermobondingverfahren werden die Fasern mittels Hitze und Druck zwischen beheizten Kalanderrollen verschmolzen. Das Ergebnis sind in beiden Fällen weiche, homogene Vliesstoffe, die ideal und für technische Anwendungen geeignet sind. Das Thermofusionsverfahren eignet sich aufgrund des fehlenden Drucks besser für Polsterschichten.

Bei der Stützschicht oder Oberschicht 2 kann es sich um ein Thermobondvlies handeln. Das Thermobondvlies weist dabei vorzugsweise lediglich eine geringe Dehnfähigkeit bei gleichzeitig gewünschter Steifigkeit auf. Somit können bei einer Elastifizierung des Verbundes 8 durch die elastischen Nähfäden 3 unterschiedliche Dehnbarkeiten und Elastizitätsmodule eingestellt werden, wobei Bereiche in dem Verbund 8, in denen keine elastischen Nähfäden 3 aufgebracht sind, keine oder nur die lediglich geringe Dehnbarkeit der nicht elastischen Stützschicht oder Oberschicht 2 aufweisen.

Die beiden Schichten 1, 2 werden zusammen einer Kettenwirkmaschine 4 zugeführt und mittels eines elastischen Nähfadens, der vorzugsweise aus einer Gruppe aus Baumwollspinnkreppfäden, Baumwollzwirnkreppfäden, texturierten Polyamidgarnen, texturierten Polyestergarnen, Gummifäden oder Polyurethanelastanfäden oder einer Kombination hiervon ausgewählt werden kann, miteinander verbunden.

Der elastische Nähfaden 3 kann alternativ auch als Kettfaden bezeichnet sein. Der Faden verläuft dabei in Maschinenrichtung der Kettenwirkmaschine und nicht quer hierzu.

Das fertig vernähte Flächengebilde des elastischen Textils 9 weist stets eine optimierte Dehnung auf, wobei besonders bevorzugt vorgesehen sein kann, dass die maximale Dehnbarkeit des elastischen Textils 9, die einer vorgegebenen optimalen Dehnbarkeit entspricht, und eine hierüber hinausgehende Dehnung des elastischen Textils 9 durch eine Dehnungsschwelle begrenzt ist. Auf diese Weise kann die Anlegersicherheit signifikant erhöht werden, da es auch für nicht geübte Anwender möglich ist, das elastische Textil 9 maximal bis zur Dehnungsschwelle zu dehnen, wobei dann nicht nur die maximale Dehnbarkeit, sondern zugleich auch die optimale Dehnung und damit der optimale Kompressionsdruck erreicht ist und in diesem maximal gedehnten Zustand das elastische Textils 9 sowie ein daraus hergestelltes medizinisches Bekleidungsstück anzulegen.

Um eine optimale Polsterwirkung zu erzielen, kann es bevorzugt vorgesehen sein, dass die Dicke der Basisschicht 1 oder Polsterschicht 0,3-12 mm, bevorzugt 0,4-6 mm und weiter bevorzugt 0,5-3 mm, besonders bevorzugt 0,6-1,2 mm beträgt.

Aufgrund der Verbindung mittels eines Nähwirkverfahrens werden die beiden Schichten 1, 2 im entspannten Zustand, nachdem das vernähte Flächengebilde des Verbundes 8 mittels Zuschneiden als Zuschnitt 11 zum medizinischen Bekleidungsstück 10 weiterverarbeitet wurde, so in Wellen gelegt, dass eine unregelmäßige Oberfläche des medizinischen Bekleidungsstücks 10 entsteht. Aufgrund dieser unregelmäßigen Oberfläche wird neben der primären Funktion als Ausgleichslage des medizinischen Bekleidungsstücks 10 zur Kompressionsbehandlung eines Fuß- und Beinbereichs und der sekundären Funktion der regulierbaren Dehnbarkeit und damit erhöhten Anlegesicherheit auch erreicht, dass durch die Wellen ein Muster auf der Oberfläche entsteht aus Materialerhebungen und Materialvertiefungen, dass auch im maximal gedehnten Zustand nicht vollständig aufgehoben ist, so dass in der Therapie hierdurch zusätzlich ein Massage- bzw. Drainageeffekt entsteht.

Zur Aufbringung eines Ruhedrucks kann darüber hinaus vorgesehen sein, das erfindungsgemäße medizinische Bekleidungsstück 10 mit einer weiteren Kompressionsschicht zu kombinieren.

Als weitere Kompressionsschicht für den Ruhedruck wird bevorzugt eine elastische, kohäsiv haftende Vliesbinde vom Typ 752, Handelsname NOWOPRESS 752, Hersteller: Karl Otto Braun GmbH & Co. KG, Wolfstein, Deutschland als Kompressionsbinde mit Langzugeigenschaften verwendet, wobei das Grundtextil durch Übernähen eines starren Polypropylenvliesstoffs mit dauerelastischen Elastanfäden in Längsrichtung unter Anwendung der Nähwirktechnik MALIWATT erhalten wurde. Die Nähwirktechnik MALIWATT ist in Malimo Nähwirktechnologie, Ploch, Böttcher, Scharch, VEB Fachbuchverlag Leipzig, 1978, 1. Auflage beschrieben. Diese Bindenlage ist auf beiden Oberflächen mit einem kohäsiv haftenden Klebemittel auf Basis Polyisopren-Kautschuk beschichtet, um den gewünschten kohäsiven Hafteffekt zu erreichen. Die Bindenlage LS (Typ 752) ist dabei wie folgt aufgebaut: 60 % Polypropylen ,12 % Elastan, 28 % IRKautschuk Grund-Vlies: PP-Spunbondvlies , 35 g/qm , thermisch geprägt Nähfaden 133 dtex Elastan (DORLASTAN, BAYER) Nähfadendichte 45 Fäden je 10 cm Breite Nähfaden-Stichlänge, Bindung 3 mm, offene Franse Flächengewicht gedehnt 59 g/qm Elastizität in Längsrichtung (Kettrichtung) Dehnbarkeit / Rückzug nach DIN 61632 160 % / 99 % Haftkraft Seite A/B 60 cN/cm.

Es ist also ein Verfahren zur Herstellung des elastischen Textils 9 vorgesehen, das die folgenden Schritte aufweist. Zunächst wird eine Basisschicht 1 und eine Oberschicht 2 bereitgestellt und eine Maschinenrichtung MD festgelegt. Ferner werden mindestens zwei Abschnitte festgelegt, deren Abschnittsgrenzen parallel zur Maschinenrichtung MD verlaufen. Ferner werden elastische Fäden 3 auf die Basisschicht 1 aufgelegt, wobei in den jeweiligen Abschnitten K1, K2, K3 eine unterschiedliche Anzahl von elastischen Fäden 3 aufgelegt werden, so dass sich die Fadendichte in den Abschnitten unterscheidet und/oder wobei in den jeweiligen Abschnitten elastische Fäden 3 mit unterschiedlicher Feinheit aufgelegt werden. Danach erfolgt ein Verbinden der Basisschicht 1 und der Oberschicht 2 durch Vernagelung oder durch Aufschmelzen, so dass das elastische Textil 9 in seiner Maschinenquerrichtung MCD Abschnitte K1, K2, K3 mit unterschiedlicher Dehnbarkeit und/oder Elastizitätsmodul aufweist.

Durch das oben beschriebene Herstellungsverfahren wird also erfindungsgemäß ein elastisches Textil 9 für medizinische Zwecke erzeugt, das einen Verbund 8 aus einer Polsterschicht oder Basisschicht 1 und einer Stützschicht oder Oberschicht 2 aufweist, wobei die beiden Schichten 1, 2 im ungedehnten Zustand mittels Nähwirkverfahren über in Maschinenrichtung MD laufende und in Maschinenquerrichtung MCD jeweils voneinander beabstandete elastische Nähfäden 3 miteinander verbunden sind. Der Verbund 8 ist in Maschinenquerrichtung MCD des Nähwirkverfahrens in mindestens zwei unterscheidbare Abschnitte K1, K2, K3 unterteilt, wobei der erste Abschnitt K1 und der zweite Abschnitt K2 unterschiedliche Dehnbarkeiten aufweisen.

Optional kann noch ein dritter Abschnitt K3 mit geringer Dehnbarkeit vorgesehen werden, der, wie weiter unten noch beschrieben werden wird, einen Brückenabschnitt 16 eines medizinischen Bekleidungsstücks 10 bildet. Die geringe Dehnbarkeit kann beispielsweise durch Weglassen von elastischen Nähfäden 3 (sodass die Dehnbarkeit des Abschnitts K3 des Verbundes 8 im Wesentlichen der Dehnbarkeit der Stützschicht oder Oberschicht 2 entspricht) oder durch Vorsehen von elastischen Nähfäden 3 mit hoher Fadendichte und/oder niedriger Feinheit in diesem Abschnitt K3 erreicht werden. Die Basisschicht 1 und/oder die Oberschicht 2 kann ein Vliesmaterial umfassen. Die Basisschicht 1 kann eine Dicke im Bereich zwischen 0,6 und 1,2 mm und/oder die Oberschicht 2 kann eine Dicke im Bereich zwischen 0,6 und 1,2 mm aufweisen. Die elastischen Nähfäden 3 können thermoplastische Elastomergarne und/oder Multifilamente umfassen, die über die Basisschicht 1 gelegt und an der Oberschicht 2 durch eine Elastomerschmelze befestigt sind.

In Fig. 2 ist die Kettenwirkmaschine 4 in Draufsicht gezeigt, wobei die elastische Nähfaden 3 in Maschinenrichtung MD in unterschiedlichen Abschnitten K1, K2, K3 unterschiedliche Feinheiten und/oder Fadendichten aufweisen und dem Vliesmaterial zugeführt und damit verbunden werden. So können unterschiedliche Elastizitäten oder Dehnungseigenschaften in den unterschiedlichen Abschnitten K1, K2, K3 des elastischen Textils 9 erzeugt werden. Die drei gezeigten Abschnitte K1, K2, K3 mit einer hohen Fadendichte in K1, einer niedrigen Feinheit in K3, und einer niedrigen Fadendichte in K2 sind lediglich als Beispiel anzusehen. So ist das elastische Textil 9 nicht auf drei Abschnitte K1, K2, K3 festgelegt. Vielmehr können auch lediglich zwei Abschnitte K1, K2 vorgesehen sein, wie beispielsweise in Fig. 2 gezeigt. Es können jedoch auch vier oder fünf verschiedene Abschnitte mit unterschiedlichen Dehnungseigenschaften vorgesehen sein.

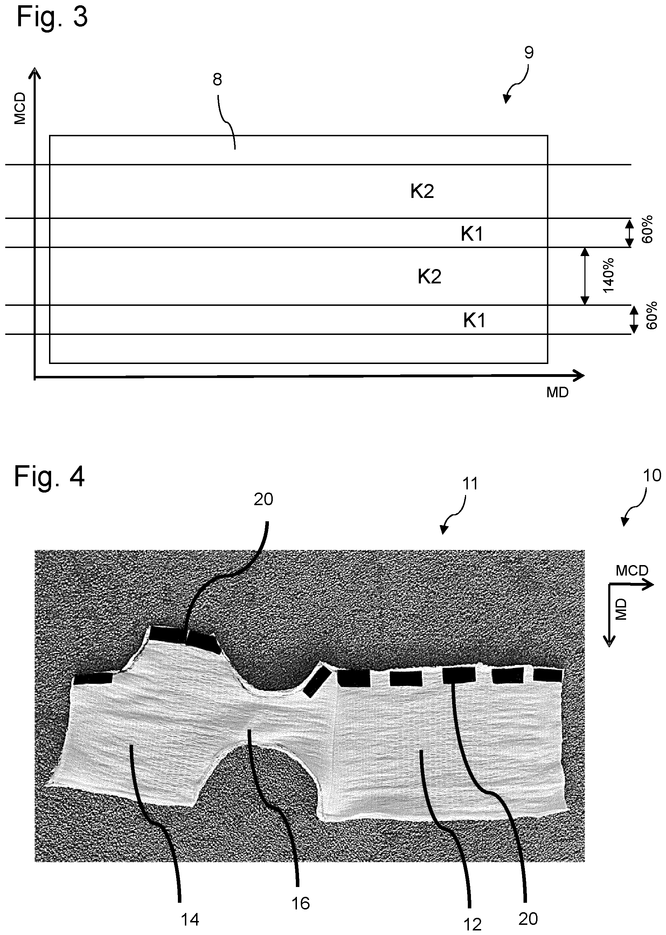

Für die Herstellung des medizinischen Bekleidungsstücks 10 können gemäß einem Ausführungsbeispiel der Erfindung drei unterschiedliche Abschnitte des elastischen Textils 9 in Maschinenquerrichtung (siehe Fig. 4 bis 7) vorgesehen sein, wobei der erste Abschnitt K1 des medizinischen Bekleidungsstücks 10 in Maschinenquerrichtung MCD eine hohe Elastizität für den Beinbereich 12, der zweite Abschnitt K2 des medizinischen Bekleidungsstücks 10 in Maschinenquerrichtung MCD eine niedrige Elastizität für den Fußbereich 14 und der dritte Abschnitt K3 des medizinischen Bekleidungsstücks 10 in Maschinenquerrichtung MCD keine oder nur eine geringe Elastizität für den Fersenbereich 16 aufweist.

Erfindungsgemäß werden also bei dem medizinischen elastischen Textil 9 unterschiedliche Bereiche in Maschinenquerrichtung MCD mit einer unterschiedlichen Dehnbarkeit versehen. Durch die vorliegende Gestaltung können gegenüber einem Textil, wie es normalerweise in der Kompressionstherapie eingesetzt wird, das über seine gesamte Länge in Maschinenquerrichtung MCD dieselben Eigenschaften aufweist, die sich aus unterschiedlichen Geometrien der umwickelten Bereiche, dem Anlegeverhalten und der Kompressionswirkung ergebenden Unterschiede flexibel berücksichtigt werden. Z. B. können elastische Textile erzeugt werden, die in ihrer Längsrichtung in Maschinenquerrichtung MCD an die unterschiedlichen Bereiche der unteren Extremität, insbesondere an den Fuß- und Beinbereich angepasste Eigenschaften aufweisen, durch die Vorsehung verschiedener Fadendichten und Feinheiten der elastischen Nahtfäden 3 in Maschinenrichtung MD.

Dabei kann über die Variation der elastischen Nahtfadendichte das Flächengewicht des Textils 9 sowie die Dehnungsfähigkeit und Kompressionskraft eingestellt werden, wobei vorgesehen sein kann, dass eine Mindestkompressionskraft über das gesamte elastische Textil 9 gewährleistet ist.

Insbesondere sind die Eigenschaften des elastischen Textils 9 hinsichtlich der drei vorgenannten Parameter über die Länge in Maschinenquerrichtung MCD des elastischen Textils 9 variabel einstellbar und individuell anpassbar. Hierdurch kann ein verbessertes Anlegeverhalten erreicht werden und der Kompressionsdruck über die Länge der zu umschließenden unteren Extremität partiell eingestellt werden. Es ist dabei denkbar, auf Basis von Vermessungen der unteren Extremität das elastische Textil 9 und den Zuschnitt 11 des elastischen Textils 9 des medizinischen Bekleidungsstücks 10 individuell für jeden Patienten anzupassen. Somit kann also erfindungsgemäß ein maßgeschneidertes medizinisches Bekleidungsstück 10 durch Variation der Form und der Maße des Zuschnitts 11 sowie der elastischen unterschiedlichen Eigenschaften der Abschnitte 12, 14, 16 des medizinischen Bekleidungsstücks 10 erzeugt werden.

Dabei ist vorgesehen, dass mit der Variation der Anzahl der elastischen Nahtfäden 3 in einem die Nahtfadendichte, also die Anzahl der Fäden pro Zentimeter Textillänge in Maschinenquerrichtung MCD variiert werden. Gemäß einem Ausführungsbeispiel der Erfindung ist die elastische Nahtfadendichte zweier in Maschinenquerrichtung MCD benachbarter Abschnitte K1, K2 unterschiedlich.

Der zweite Abschnitt K2 kann eine Dehnbarkeit in Maschinenrichtung von 100 % - 180 % mit einem Elastizitätsmodul von kleiner 6 kPa und/oder der erste Abschnitt K1 kann eine Dehnbarkeit in Maschinenrichtung von 20 - 95 % mit einem Elastizitätsmodul von kleiner 40 kPa aufweisen. Hierbei kann der erste Abschnitt K1 und der zweite Abschnitt K2 in Maschinenquerrichtung MCD direkt aufeinander folgend abwechselnd angeordnet sein, wie in Fig. 3 gezeigt. Der erste Abschnitt K1 und der zweite Abschnitt K2 können jedoch in Maschinenquerrichtung MCD nicht direkt benachbart angeordnet sein, wobei ein Zwischenabschnitt K3 vorgesehen ist, wie in Fig. 2 gezeigt. Der zweite Abschnitt K1 kann insbesondere eine Dehnbarkeit in Maschinenrichtung von 140 % +/- 10 % aufweisen. Der erste Abschnitt K1 kann eine Dehnbarkeit in Maschinenrichtung von 60 % +/- 10 % aufweisen.

Im ersten und im zweiten Abschnitt K1, K2 können die die elastischen Nähfäden 3 mit jeweils unterschiedlichen Feinheiten und/oder mit in Maschinenquerrichtung jeweils unterschiedlichen Fadendichten aufgebracht sein. Im ersten und/oder im zweiten Abschnitt K1, K2 können die elastischen Nähfäden 3 innerhalb eines jeweiligen Abschnitts K1, K2 mit unterschiedlichen Feinheiten und/oder mit in Maschinenquerrichtung unterschiedlichen Fadendichten aufgebracht sein. Der Verbund 8 kann zumindest zwei elastische Nähfäden 3, insbesondere drei elastische Nähfäden 3 mit unterschiedlicher Feinheit aufweisen.

Die elastischen Nähfäden 3 können im ersten Abschnitt K1 eine Feinheit im Bereich zwischen 50 dtex und 500 dtex, oder zwischen 100 dtex und 400 dtex, oder zwischen 100 dtex und 300 dtex, oder zwischen 100 dtex und 250 dtex, oder zwischen 100 und 200 dtex, oder zwischen 100 und 150 dtex aufweisen. Die elastischen Nähfäden 3 können im zweiten Abschnitt K2 eine Feinheit im Bereich zwischen 50 dtex und 500 dtex, oder zwischen 100 dtex und 500 dtex, oder zwischen 200 dtex und 500 dtex, oder zwischen 200 dtex und 400 dtex, oder zwischen 250 dtex und 350 dtex aufweisen. Insbesondere können die elastischen Nähfäden 3 im ersten Abschnitt K1 und im zweiten Abschnitt K2 eine Feinheit von 135 dtex aufweisen, da eine Einstellung von unterschiedlichen Feinheiten optional ist.

Die elastischen Nähfäden 3 können im ersten Abschnitt K1 mit einer Fadendichte im Bereich zwischen 50 und 500 Fäden pro 10 cm, oder zwischen 100 und 400 Fäden pro 10 cm, oder zwischen 150 und 350 Fäden pro 10 cm, oder zwischen 150 und 300 Fäden pro 10 cm aufgebracht sein. Die elastischen Nähfäden 3 können im zweiten Abschnitt K2 mit einer Fadendichte im Bereich zwischen 50 und 500 Fäden pro 10 cm, oder zwischen 50 und 400 Fäden pro 10 cm, oder zwischen 100 und 300 Fäden pro 10 cm, oder zwischen 100 und 200 Fäden pro 10 cm aufgebracht sein.

Weiterhin kann die Nahtfadendichte des ersten Abschnitts K1 mit höherer Nahtfadendichte zu der Nahtfadendichte des zweiten Abschnitts K2 mit niedrigerer Nahtfadendichte ein Verhältnis von 3:1, oder von 2,5:1, oder von 2:1 aufweisen.

Ferner kann der mittlere Fadenabstand im ersten Abschnitt K1 in einem Bereich von 0,5 mm bis 1 mm, oder in einem Bereich von 0,1 mm bis 10 mm, oder in einem Bereich von 0.5 mm bis 8 mm, oder in einem Bereich von 1 mm bis 7 mm, oder in einem Bereich von 1 mm bis 6 mm, oder in einem Bereich von 1 mm bis 5 mm, oder in einem Bereich von 1 mm bis 4 mm, oder in einem Bereich von 1 mm bis 3 mm, oder in einem Bereich von 1 mm bis 2 mm liegen. Im zweiten Abschnitt K2 kann der mittlere Fadenabstand in einem Bereich von 4 mm bis 4,5 mm, oder in einem Bereich von 3 mm bis 5 mm, oder in einem Bereich von 3 mm bis 5,5 mm, oder in einem Bereich von 2 mm bis 6 mm, oder in einem Bereich von 1 mm bis 15 mm, oder in einem Bereich von 2 mm bis 12 mm, oder in einem Bereich von 3 mm bis 10 mm, oder in einem Bereich von 4 mm bis 8 mm, oder in einem Bereich von 5 mm bis 8 mm, oder in einem Bereich von 6 mm bis 8 mm liegen.

Dies ist jedoch nur beispielhaft und weitere Feinheiten oder Fadenstärken und Fadendichten oder Abstände sind möglich. Weiterhin können die Fäden 3 eine Fadenvorspannung von größer 50 %, oder größer 100 %, oder größer 150 %, oder größer 200 %, oder größer 250 %, oder größer 300 %, oder größer 350%, oder größer 400 %, oder größer 450 %, oder größer 500 % und/oder kleiner als 600 % aufweisen.

Die folgende Tabelle zeigt beispielhaft sämtliche Parameter eines erfindungsgemäßen elastischen Textils 9 für medizinische Zwecke, das auch für das medizinische Bekleidungsstück 10 gemäß der Erfindung eingesetzt werden kann:

| | **Abschnitt 1 (Bein)** | **Abschnitt 2 (Fuß)** |
|---|---|---|
| **Dehnbarkeit** | 20 - 95 % | 100% - 180 % |
| | Insbesondere 60 % | Insbesondere 140 % |
| **Functional Stretch** | 30 - 50 % | 50 - 100 % |
| | | |
| | | |
| **Young's Modul bei maximaler Spannung von 8 N/cm (Elastizitätsmodul)** | ≤ 40 kPa | o≤ 6 kPa |
| **Therapeutischer Druck** | 2-6 kPa | 2 -3 kPa |
| | | |
| | | |
| **Fadendichte** | höher | geringer |
| **Rückstellfähigkeit** | höher | geringer |
| | | |
| **Feinheit (optional)** | 135 dtex | 135 dtex |

Das Textil 9 kann weitere Lagen aufweisen, insbesondere können klebende Lagen, die insbesondere in Form einer klebenden Beschichtung auf dem Textil des Bandmaterials aufgebracht sind, vorgesehen sein. Die klebende Beschichtung kann dabei sowohl adhäsiver als auch rein kohäsiver Natur sein.

Gemäß den obigen Parametern ist der Verbund 8 in der Lage, einer Belastung in Maschinenrichtung MD von mindestens 2 kPa standzuhalten, ohne sich während der Belastung in der Breite in Maschinenquerrichtung MCD zu verengen. Dabei sind, wie noch genauer beschrieben werden wird, der erste und zweite Abschnitt K1, K2 mit unterschiedlicher Dehnbarkeit in Maschinenquerrichtung MCD für das Anlegen des Verbundes 8 an unterschiedliche anatomische Bereiche von Gliedmaßen, insbesondere für Bein, Ferse und Fuß vorgesehen. Der erste Abschnitt K1 ist dabei für das Anlegen des Verbundes 8 an einen Beinbereich mit einem therapeutischen Zielgrenzdruck von 2-8 kPa und der zweite Abschnitt K2 für das Anlegen des Verbundes 8 an einen Fußbereich mit einem therapeutischen Zielgrenzdruck von 2-3 kPa vorgesehen.

In den Fig. 3 und Fig. 4 ist ein elastisches Textil 9 für medizinische Zwecke sowie ein medizinisches Bekleidungsstück 10 gemäß einem Ausführungsbeispiel der Erfindung gezeigt.

Das medizinische Bekleidungsstück 10 besteht aus einem genähten Vliesstoff, einem Legewerk, bei dem thermoplastische Urethan-Multifilamentgarne mit einer Feinheit von 133, 156 und 320 dtex systematisch über die Oberfläche des Grundgewebes gelegt und entweder durch eine Elastomerschmelze oder durch Nähte an der Oberfläche des Obergewebes befestigt werden, so dass mindestens zwei Abschnitte K1, K2 mit unterschiedlicher Dehnbarkeit entstehen. Der Verbund 8 kann also ein genähtes Vlies oder ein Gelege sein, bei dem thermoplastische Urethan-Multifilamentgarne mit einer Feinheit von 133, 156 und 320 dtex systematisch auf die Oberfläche des Grundgewebes gelegt und entweder durch eine Elastomerschmelze oder durch Nähte an der Oberfläche des Obergewebes befestigt werden, um mindestens zwei Abschnitte K1, K2 mit unterschiedlicher Dehnbarkeit zu bilden.

Es besteht ferner aus einem elastischen Textil 9, das in der Maschinenquerrichtung MCD in zwei unterscheidbare Abschnitte K1, K2 unterteilt ist, von denen der erste durch eine Dehnbarkeit von 60 % und der zweite durch eine Dehnbarkeit von 140 % für den Beinabschnitt bzw. Fußabschnitt gekennzeichnet ist, wenn er als Fuß- und Beinkleidungsstück hergestellt wird. Der Verbund 8 kann also aus einem elastischen Textil bestehen, das in der Maschinenquerrichtung in zwei unterscheidbare Abschnitte unterteilt ist, von denen der erste durch eine Dehnbarkeit von 60 % und der zweite durch eine Dehnbarkeit von 140 % für den Beinabschnitt bzw. den Fußabschnitt gekennzeichnet ist, wenn er als Fuß- und Beinkleidungsstück hergestellt wird.

Erfindungsgemäß ist also ein Kompressionskleidungsstück 10 mit mindestens einem Fußteil 14 und einem Beinteil 12 vorgesehen, das den Gefäßfluss vom Fuß bis unterhalb des Knies kontrollieren kann, das auch in der Lage ist, den gesamten plantaren Venenplexus des Fußes ohne Unterbrechung durch den Fersenbereich 16 bis zum Bereich des Malleolus gastrocnemius zu überdecken, und das mit unterschiedlicher Ausdehnung des gleichen oder mehrerer Strukturaufbauten des Elastomers der Gesamtstruktur Elastizität verleiht.

Wenn der Verschluss 20 des Bekleidungsstücks bis zu einer maximalen Spannung von 34 cN/cm gedehnt wird, um das Bekleidungsstück 10 am Fuß zu schließen, wird ein lokaler Schnittstellendruck von etwa 13 mmHg (etwa 1,7 kPa) auf den Fuß des Patienten ausgeübt. Um den erforderlichen Druck zu erreichen, muss das Material je nach seinen elastischen Eigenschaften beispielsweise mit einer Dehnung von 26 % angelegt werden.

Bei der konventionellen Kompressionstherapie für Gefäßerkrankungen wird aufgrund des hydrostatischen Gesetzes der Strömungsmechanik am Fuß ein geringerer Druck als am Bein verwendet. Daher kann die Anwendung eines Drucks von 13 mmHg in Ruhestellung gegeben sein, und auch dann wirksam sein, wenn die Person steht. Wenn der Verschluss 20 des Bekleidungsstücks 10 bis zu einer maximalen Spannung von 107 cN/cm gedehnt wird, um das Bekleidungsstück 10 am Malleolus gastrocnemius zu schließen, wird voraussichtlich ein lokaler Schnittstellendruck von etwa 35 mmHg (etwa 5 kPa) auf das Bein des Patienten ausgeübt. Um den erforderlichen Druck zu erreichen, muss das Material je nach seinen elastischen Eigenschaften beispielsweise mit einer Dehnung von 44 % angelegt werden.

Durch die Erfindung wird also ein medizinisches Bekleidungsstück 10 geschaffen, das optimal gestaltet ist und das darauf abzielt, Kontrollmechanismen festzusetzen, die den physiologischen Fluss während der Kompressionstherapie unterstützen. Die verschiedenen im Bekleidungsstück 10 ausgebildeten Dehnbarkeiten haben unterschiedliche Elastizitätskoeffizienten. Typischerweise ist der Elastizitätskoeffizient des Fußteils 14 des Bekleidungsstücks 10 niedriger als der Elastizitätskoeffizient des Beinteils 12.

Wenn der Verschluss 20 des Bekleidungsstücks 10 bis zu einer maximalen Spannung von 210 cN/cm gedehnt wird, um das Bekleidungsstück 10 am Malleolus gastrocnemius zu schließen, wird ein lokaler Schnittstellendruck von etwa 67 mmHg (ca. 10 kPa) auf das Bein des Patienten ausgeübt. Um den erforderlichen Druck zu erreichen, muss das Material je nach seinen elastischen Eigenschaften beispielsweise mit einer Dehnung von 47 % angelegt werden.

Das erfindungsgemäße medizinische Bekleidungsstück 10 besteht aus einem mindestens zweiteiligen elastischen Textil 9, das ein Kompressionsbekleidungsstück 10 mit einem Fuß-, einem Fersen- und einem Beinteil bildet, die untrennbar miteinander verbunden sind und einzeln verschlossen werden können, so dass der Anpressdruck nur im Fuß- und im Beinteil eingestellt werden kann. Ferner besteht es aus einem medizinischen Kompressionsbekleidungsstück 10 mit einem Fußteil 14 mit einer funktionellen Dehnung von 50-100 %, was zu einem therapeutischen Druck von 2-3 kPa führt. Darüber hinaus besteht es aus einem Kompressionsbekleidungsstück 10 mit einem Fußteil 14, dessen Elastizitätsmodul ≤ 6 kPa ist, bei 40 % Dehnung und einer spezifischen Spannung von 1 N/cm. Die Erfindung besteht ferner aus einem Kompressionsbekleidungsstück, dessen Beinteil 12 für einen therapeutischen Druck von 2-8 kPa mit einer Dehnung von 40-45 % bei phlebolytischer Insuffizienz ausgelegt ist. Ferner ist erfindungsgemäß ein Kompressionsbekleidungsstück 10 vorgesehen, dessen Beinteil 12 ein Elastizitätsmodul von ≤ 40 kPa bei einer maximalen Spannung von 8 N/cm aufweist. Hierbei besteht es aus einem Kompressionsbekleidungsstück 12, das leicht zu tragen ist und nur an zwei Stellen geschlossen wird (keine Mehrfachverschlüsse), d. h. am Fuß und am Bein.

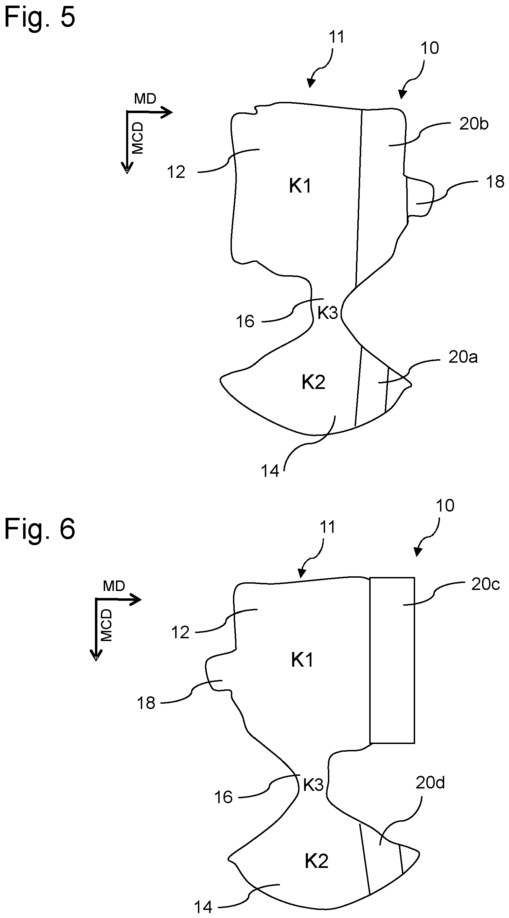

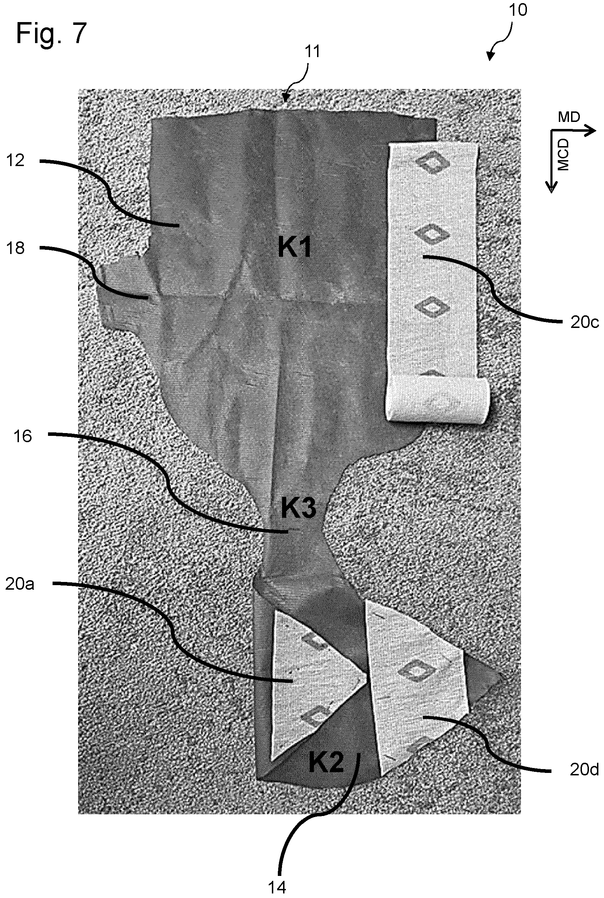

In den Fig. 5 bis Fig. 7 sind die Außenseite (Fig. 5) sowie die Innenseite (Fig. 6) sowie eine Innenseite mit eingeklapptem Verschlusselement (Fig. 7) eines medizinischen Bekleidungsstücks 10 gemäß einem Ausführungsbeispiel der Erfindung gezeigt.

Das medizinische Bekleidungsstück 10 ist zum Ausüben eines Kompressionsdrucks auf eine untere Extremität angepasst. Es weist einen einteiligen Zuschnitt 11 eines in Maschinenrichtung MD elastischen Textils 9 mit einem ersten und einem zweiten Abschnitt K1, K2 in Maschinenquerrichtung MCD auf. Hierbei ist der erste Abschnitt K1 mit einer ersten Elastizität zum Anlegen an das Bein und der zweite Abschnitt K2 ist mit einer zweiten Elastizität zum Anlegen an einen Fuß vorgesehen.

Des Weiteren sind Befestigungsmittel 20 vorgesehen, die dazu angepasst sind, durch überlappende Verbindung von entgegengesetzten Enden des Zuschnitts 11 in Maschinenrichtung MD das Bekleidungsstück 10 mittels der Verschlusselemente 20 die untere Extremität umschließend lösbar zu verschließen. Erfindungsgemäß ist das Bekleidungsstück 10 dazu angepasst, einen ersten Kompressionsdruck auf das Bein und einen zweiten Kompressionsdruck auf den Fuß auszuüben, wobei der erste Kompressionsdruck höher als der zweite Kompressionsdruck ist. Der Zuschnitt ist dabei vorzugsweise aus einem elastischen Textil, wie es oben beschrieben wurde, gefertigt.

Der Zuschnitt kann ferner, wie in den Fig. 5 und 6 gezeigt, einen dritten Abschnitt K3 zum Anlegen an die Ferse aufweisen, der eine dritte Elastizität aufweist. Der erste und der zweite Abschnitt K1, K2 weisen dabei erste bzw. zweite Befestigungsmittel auf, so dass das elastische Textil 9 um das Bein bzw. den Fuß gelegt und auf sich selbst befestigt werden kann. Der dritte Abschnitt K3 weist hierbei keine oder eine sehr geringe Elastizität in Maschinenrichtung MD auf und ist dazu vorgesehen, einen Brückenabschnitt 16 zwischen dem ersten und zweiten Abschnitt K1, K2 auszubilden, um das Anlegen des Bekleidungsstückes 10 an Fuß und Bein zu erleichtern.

Der zweite Abschnitt K2, 14 besitzt vorzugsweise ein Elastizitätsmodul von ≤ 6 kPa, bei 40% Dehnung und bei einer spezifischen Spannung von 1 N/cm. Der erste Abschnitt ist für einen Therapiedruck von 2-3 kPa mit 40-45% ausgelegt. Der erste Abschnitt weist ein Elastizitätsmodul von ≤ 40 kPa bei einer maximalen Spannung von 8 N/cm aufweist Vorzugsweise sind im ersten und zweiten Abschnitt K1, K2 jeweils nur ein Befestigungsmittel 20 vorgesehen. Das Befestigungsmittel 20 umfasst dabei Verschlusselemente 20a, 20b, 20c, 20d mit klebender Beschichtung, Klettverschlüsse oder Reißverschlüsse. Bei dem Befestigungsmittel 20 kann es sich um Haken- und Stapelverschlüsse handeln. Das Befestigungsmittel 20 kann auch eine kohäsive Beschichtung sein, die auf der Innenseite des Fußes und auf der Außenseite des Fußes angebracht werden kann. Ferner können Klettverschlüsse (Velcro), adhäsive Beschichtung oder auch Reißverschlüsse vorgesehen sein, wobei gegebenenfalls mehrere Reihen versetzt angeordnet sein können. Die Anordnung der Verschlüsse 20 ist hierbei vorzugsweise durchgehend in Längsrichtung oder in Maschinenquerrichtung MCD des medizinischen Bekleidungsstücks 10 gemäß einem Ausführungsbeispiel der Erfindung. Der erste und/oder zweite Abschnitt K1, K2 kann auch Indikatoren oder Markierungen (nicht gezeigt) für die Verschlusselemente 20 aufweisen, um einen definierten Kompressionsdruck bei Anlegen des Bekleidungsstücks 10 auf Bein und Fuß auszuüben.

Wie weiter in den Fig. 5 und 6 gezeigt, kann der erste Abschnitt K1 des Zuschnitts 11 eine rechteckige Form und der zweite Abschnitt K2 des Zuschnitts 11 kann eine "American Football"-Form oder eine Zitronen-Form aufweisen. Der dritte Abschnitt K3 des Zuschnitts 11 kann als Brückenabschnitt 16 zwischen erstem und zweitem Abschnitt 12, 14 sanduhrförmig ausgebildet sein, um eine Zugkraft von dem ersten Abschnitt 12 zu dem zweiten Abschnitt 14 breitflächig zu übertragen.

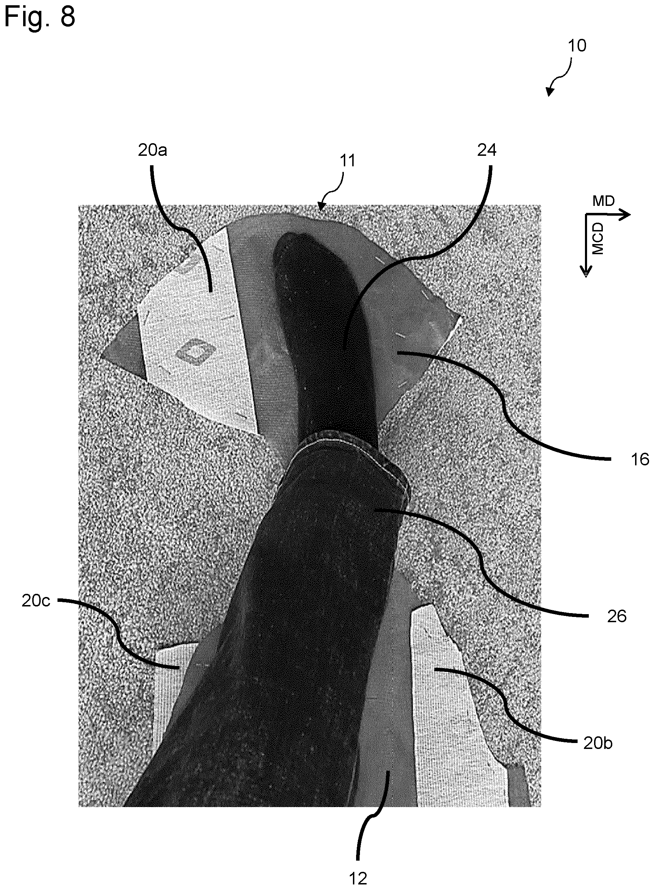

In der Fig. 8 ist ein Verfahren zum Anlegen eines medizinischen Bekleidungsstücks 10 illustriert, das die folgenden Schritte aufweist: Auflegen des Zuschnitts 11 des medizinischen Bekleidungsstücks 10 auf einen flachen Untergrund; Betreten und Fixieren des zweiten Abschnitts 14 durch einen Benutzer, Verschließen des Bekleidungsstücks 10 im Fußbereich durch den Benutzer mit einem definierten zweiten Kompressionsdruck; Greifen des proximalen Endes im ersten Abschnitt 12 durch einen Benutzer, während er noch auf dem zweiten Abschnitt 14 steht; Ziehen des ersten Abschnitts 12 nach oben in Richtung des Rumpfes des Benutzers, wobei aufgrund der Einteiligkeit des Zuschnitts 11 ein Kraftschluss zwischen erstem und zweitem Abschnitt 12, 14 zustande kommt, sodass sich der erste Abschnitt 12 in Beinrichtung und Maschinenquerrichtung MCD des Zuschnitts 11 lokal definiert das Bein umschließt; Verschließen des Bekleidungsstücks 10 im Beinbereich durch den Benutzer mit einem definierten ersten Kompressionsdruck. Hierbei dehnt sich der dritte Abschnitt 16, der an der Achillessehne der Ferse, Calcaneus, geführt wird, nicht bei der Anwendung des medizinischen Bekleidungsstücks 10.

Erfindungsgemäß ist auch eine Verwendung des medizinischen Bekleidungsstücks bei phlebo-lymphatischer Insuffizienz beansprucht.

Es folgt eine Liste von Ausführungsbeispielen gemäß der Erfindung:
1. Elastisches Textil (9) für medizinische Zwecke, mit
   - einem Verbund (8) aus einer Basisschicht (1) und einer Oberschicht (2), wobei die beiden Schichten (1,2) im ungedehnten Zustand mittels Nähwirkverfahren über in Maschinenrichtung (MD) laufende und in Maschinenquerrichtung (MCD) jeweils voneinander beabstandete elastische Nähfäden (3) miteinander verbunden sind, **dadurch**
   **gekennzeichnet, dass**
   der Verbund (8) in Maschinenquerrichtung (MCD) des Nähwirkverfahrens in mindestens zwei unterscheidbare Abschnitte (K1, K2) unterteilt ist, wobei der erste Abschnitt (K1) und der zweite Abschnitt (K2) unterschiedliche Dehnbarkeiten in Maschinenrichtung (MD) aufweisen.
2. Elastisches Textil (9) nach Punkt 1, **dadurch gekennzeichnet, dass** die Basisschicht (1) und/oder die Oberschicht (2) ein Vliesmaterial umfassen.
3. Elastisches Textil (9) nach einem der vorstehenden Punkte, **dadurch gekennzeichnet, dass** die Basisschicht (1) eine Dicke im Bereich zwischen 0,6 und 1,2 mm und/oder die Oberschicht (2) eine Dicke im Bereich zwischen 0,6 und 1,2 mm aufweist.
4. Elastisches Textil (9) nach einem der vorstehenden Punkte, **dadurch gekennzeichnet, dass** die elastischen Nähfäden (3) thermoplastische Elastomergarne und/oder Multifilamente umfassen, die über die Basisschicht (1) gelegt und an der Oberschicht (2) durch eine Elastomerschmelze befestigt sind.
5. Elastisches Textil (9) nach einem der vorstehenden Punkte, **dadurch gekennzeichnet, dass** die elastischen Nähfäden (3) aus einer Gruppe von Baumwoll-Spinnkreppfäden, Baumwoll-Zwirnkreppfäden, texturierten Polyamidgarnen, texturierten Polyestergarnen, Gummifäden oder Polyurethan-Elastanfäden oder einer Kombination hieraus ausgewählt werden.
6. Elastisches Textil (9) nach einem der vorstehenden Punkte, **dadurch gekennzeichnet, dass** der erste Abschnitt (K1) eine Dehnbarkeit in Maschinenrichtung von 20 - 95 % mit einem Elastizitätsmodul von kleiner 40 kPa und/oder der zweite Abschnitt (K2) eine Dehnbarkeit in Maschinenrichtung von 100 % - 180 % mit einem Elastizitätsmodul von kleiner 6 kPa aufweist.
7. Elastisches Textil (9) nach einem der vorstehenden Punkte, **dadurch gekennzeichnet, dass** der erste Abschnitt (K1) eine Dehnbarkeit in Maschinenrichtung von 60 % +/- 10 % und/oder der zweite Abschnitt (K2) eine Dehnbarkeit in Maschinenrichtung von 140 % +/- 10 % aufweist.
8. Elastisches Textil (9) nach einem der vorstehenden Punkte, **dadurch gekennzeichnet, dass** im ersten und im zweiten Abschnitt (K1, K2) die elastischen Nähfäden (3) mit jeweils unterschiedlichen Feinheiten und/oder mit in Maschinenquerrichtung (MCD) jeweils unterschiedlichen Fadendichten aufgebracht sind.
9. Elastisches Textil (9) nach einem der vorstehenden Punkte, **dadurch gekennzeichnet, dass** im ersten und/oder im zweiten Abschnitt (K1, K2) die elastischen Nähfäden (3) innerhalb eines jeweiligen Abschnitts (K1, K2) mit unterschiedlichen Feinheiten und/oder mit in Maschinenquerrichtung (MCD) unterschiedlichen Fadendichten aufgebracht sind.
10. Elastisches Textil (9) nach einem der vorstehenden Punkte, **dadurch gekennzeichnet, dass** der Verbund (8) zumindest zwei elastische Nähfäden (3), insbesondere drei elastische Nähfäden (3) mit unterschiedlicher Feinheit aufweist.
11. Elastisches Textil (9) nach einem der vorstehenden Punkte, **dadurch gekennzeichnet, dass** die elastischen Nähfäden (3) im ersten Abschnitt (K1) eine Feinheit im Bereich zwischen 100 dtex und 200 dtex und im zweiten Abschnitt (K2) eine Feinheit im Bereich zwischen 100 dtex und 400 dtex aufweisen.
12. Elastisches Textil (9) nach einem der vorstehenden Punkte, **dadurch gekennzeichnet, dass** die elastischen Nähfäden (3) im ersten Abschnitt (K1) mit einer Fadendichte im Bereich zwischen 100 und 300 Fäden pro 10 cm und im zweiten Abschnitt (K2) mit einer Fadendichte im Bereich zwischen 100 und 300 Fäden pro 10 cm aufgebracht sind.
13. Elastisches Textil (9) nach einem der vorstehenden Punkte, **dadurch gekennzeichnet, dass** der Verbund (8) in der Lage ist, einer Belastung in Maschinenrichtung (MD) von mindestens 2 kPa standzuhalten, ohne sich während der Belastung in der Breite in Maschinenquerrichtung (MCD) zu verengen.
14. Elastisches Textil (9) nach einem der vorstehenden Punkte, **dadurch gekennzeichnet, dass** der erste und zweite Abschnitt (K1, K2) mit unterschiedlicher Dehnbarkeit in Maschinenquerrichtung (MCD) für das Anlegen des Verbundes (8) an unterschiedliche anatomische Bereiche von Gliedmaßen, insbesondre für Bein, Ferse und Fuß vorgesehen sind.
15. Elastisches Textil (9) nach einem der vorstehenden Punkte, **dadurch gekennzeichnet, dass** der erste Abschnitt (K1) für das Anlegen des Verbundes (8) an einen Beinbereich mit einem therapeutischen Zielgrenzdruck von 2-8 kPa und der zweite Abschnitt (K2) für das Anlegen des Verbundes (8) an einen Fußbereich mit einem therapeutischen Zielgrenzdruck von 2-3 kPa vorgesehen sind.
16. Elastisches Textil (9) nach einem der vorstehenden Punkte, **dadurch gekennzeichnet, dass** der Verbund (8) ein genähtes Vlies oder ein Gelege ist, bei dem thermoplastische Urethan-Multifilamentgarne mit einer Feinheit von 133, 156 und 320 dtex systematisch auf die Oberfläche des Grundgewebes gelegt und entweder durch eine Elastomerschmelze oder durch Nähte an der Oberfläche des Obergewebes befestigt werden, um mindestens zwei Abschnitte mit unterschiedlicher Dehnbarkeit zu bilden.
17. Elastisches Textil (9) nach einem der vorstehenden Punkte, **dadurch gekennzeichnet, dass** der Verbund (8) aus einem elastischen Textil besteht, das in der Maschinenquerrichtung in zwei unterscheidbare Abschnitte unterteilt ist, von denen der erste durch eine Dehnbarkeit von 60 % und der zweite durch eine Dehnbarkeit von 140 % für den Beinabschnitt bzw. den Fußabschnitt gekennzeichnet ist, wenn er als Fuß- und Beinkleidungsstück hergestellt wird.
18. Medizinisches Bekleidungsstück (10) zum Ausüben eines Kompressionsdrucks auf eine untere Extremität, mit
   - einem einteiligen Zuschnitt (11) eines in Maschinenrichtung (MD) elastischen Textils (9) mit einem ersten und einem zweiten Abschnitt (K1, K2) in Maschinenquerrichtung (MCD), wobei der erste Abschnitt (K1) mit einer ersten Elastizität zum Anlegen an das Bein vorgesehen ist und der zweite Abschnitt (K2) mit einer zweiten Elastizität zum Anlegen an einen Fuß vorgesehen ist;
   - Befestigungsmittel (20), die dazu angepasst sind, durch überlappende Verbindung von entgegengesetzten Enden des Zuschnitts (11) in Maschinenrichtung (MD) das Bekleidungsstück (10) mittels Verschlusselementen (20a, 20b, 20c, 20d) die untere Extremität umschließend lösbar zu verschließen, **dadurch gekennzeichnet, dass**
      das Bekleidungsstück (10) dazu angepasst ist, einen ersten Kompressionsdruck auf das Bein und einen zweiten Kompressionsdruck auf den Fuß auszuüben, wobei der erste Kompressionsdruck höher als der zweite Kompressionsdruck ist.
19. Medizinisches Bekleidungsstück (10) nach Punkt 18, **dadurch gekennzeichnet, dass** der Zuschnitt (11) aus einem elastischen Textil (9) gemäß einem der Punkte 1 bis 17 gefertigt ist.
20. Medizinisches Bekleidungsstück (10) nach Anspruch 18 oder 19, **dadurch gekennzeichnet, dass** der Zuschnitt (11) einen dritten Abschnitt (K3) zum Anlegen an die Ferse aufweist, der eine dritte Elastizität aufweist.
21. Medizinisches Bekleidungsstück (10) nach einem der Punkte 18 bis 20, **dadurch gekennzeichnet, dass** der erste und zweite Abschnitt (K1, K2) erste bzw. zweite Befestigungsmittel (20) aufweisen, so dass das elastische Textil (9) um das Bein bzw. den Fuß gelegt und auf sich selbst befestigt werden kann.
22. Medizinisches Bekleidungsstück (10) nach einem der Punkte 18 bis 21, **dadurch gekennzeichnet, dass** der dritte Abschnitt (K3) keine Elastizität in Maschinenrichtung (MD) aufweist und dazu vorgesehen ist, einen Brückenabschnitt (16) zwischen erstem (12) und zweitem (14) Abschnitt auszubilden, um das Anlegen des Bekleidungsstückes (10) an Fuß und Bein zu erleichtern.
23. Medizinisches Bekleidungsstück (10) nach einem der Punkte 18 bis 22, **dadurch gekennzeichnet, dass** der zweite Abschnitt (K2) ein Elastizitätsmodul von ≤ 6 kPa, bei 40% Dehnung und bei einer spezifischen Spannung von 1 N/cm aufweist.
24. Medizinisches Bekleidungsstück (10) nach einem der Punkte 18 bis 23, **dadurch gekennzeichnet, dass** der erste Abschnitt (K1) für einen Therapiedruck von 2-8 kPa mit 40-45% ausgelegt ist.
25. Medizinisches Bekleidungsstück (10) nach einem der Punkte 18 bis 24, **dadurch gekennzeichnet, dass** der erste Abschnitt (K1) ein Elastizitätsmodul von ≤ 40 kPa bei einer maximalen Spannung von 8 N/cm aufweist
26. Medizinisches Bekleidungsstück (10) nach einem der Punkte 18 bis 25, **dadurch gekennzeichnet, dass** im ersten und zweiten Abschnitt (K1, K2) jeweils nur ein Befestigungsmittel (20) vorgesehen ist.
27. Medizinisches Bekleidungsstück (10) nach einem der Punkte 18 bis 26, **dadurch gekennzeichnet, dass** das Befestigungsmittel (20) Verschlusselemente mit klebender Beschichtung, Klettverschlüsse oder Reißverschlüsse umfasst.
28. Medizinisches Bekleidungsstück (10) nach einem der Punkte 18 bis 27, **dadurch gekennzeichnet, dass** der erste und/oder der zweite Abschnitt (K1, K2) Indikatoren oder Markierungen für die Verschlusselemente (20) aufweisen, um einen definierten Kompressionsdruck bei Anlegen des Bekleidungsstücks (10) auf Bein und Fuß auszuüben.
29. Medizinisches Bekleidungsstück (10) nach einem der Punkte 18 bis 28, **dadurch gekennzeichnet, dass** der erste Abschnitt (K1) des Zuschnitts (11) eine rechteckige Form aufweist.
30. Medizinisches Bekleidungsstück (10) nach einem der Punkte 18 bis 29, **dadurch gekennzeichnet, dass** der zweite Abschnitt (K2) des Zuschnitts (11) eine "American Football"-Form oder eine Zitronen-Form aufweist.
31. Medizinisches Bekleidungsstück (10) nach einem der Punkte 18 bis 29, **dadurch gekennzeichnet, dass** der dritte Abschnitt (K3) des Zuschnitts (11) als Brückenabschnitt (16) zwischen erstem (12) und zweitem (14) Abschnitt sanduhrförmig ausgebildet ist, um eine Zugkraft von dem ersten Abschnitt (12) zu dem zweiten Abschnitt (14) breitflächig zu übertragen.
32. Verfahren zum Anlegen eines medizinischen Bekleidungsstücks (10) gemäß einem der Punkte 18 bis 31, mit den Schritten:
   - Auflegen des Zuschnitts (11) des medizinischen Bekleidungsstücks (10) auf einen flachen Untergrund;
   - Betreten und Fixieren des zweiten Abschnitts (K2) durch einen Benutzer,
   - Verschließen des Bekleidungsstücks (10) im Fußbereich durch den Benutzer mit einem definierten zweiten Kompressionsdruck;
   - Greifen des proximalen Endes im ersten Abschnitt (K1) durch einen Benutzer, während er noch auf dem zweiten Abschnitt (K2) steht;
   - Ziehen des ersten Abschnitts (K1) nach oben in Richtung des Rumpfes des Benutzers, wobei aufgrund der Einteiligkeit des Zuschnitts (11) ein Kraftschluss zwischen erstem und zweitem Abschnitt (K1, K2) zustande kommt, sodass sich der erste Abschnitt (K1) in Beinrichtung und Maschinenquerrichtung (MCD) des Zuschnitts (11) lokal definiert das Bein umschließt;
   - Verschließen des Bekleidungsstücks (10) im Beinbereich durch den Benutzer mit einem definierten ersten Kompressionsdruck.
33. Verfahren zum Anlegen eines medizinischen Bekleidungsstücks nach Punkt 32, **dadurch gekennzeichnet, dass** sich der dritte Abschnitt (K3), der an der Achillessehne der Ferse, Calcaneus, geführt wird, sich bei der Anwendung des medizinischen Bekleidungsstücks (10) nicht dehnt.
34. Verwendung des medizinisches Bekleidungsstück (10) nach einem der Punkte 18 bis 31 bei phlebo-lymphatischer Insuffizienz.
35. Verfahren zur Herstellung des elastischen Textils (9) nach einem der Punkte 1 bis 17, mit den Schritten:
   - Bereitstellen einer Basisschicht (1) und einer Oberschicht (2);
   - Festlegen einer Maschinenrichtung (MD);
   - Festlegen von mindestens zwei Abschnitten (K1, K2), deren Abschnittsgrenzen parallel zur Maschinenrichtung (MD) verlaufen;
   - Auflegen von elastischen Fäden (3) auf die Basisschicht (1), wobei in den jeweiligen Abschnitten (K1, K2) eine unterschiedliche Anzahl von elastischen Fäden (3) aufgelegt werden, so dass sich die Fadendichte in den Abschnitten (K1, K2) unterscheidet und/oder wobei in den jeweiligen Abschnitten (K1, K2) Fäden (3) mit unterschiedlicher Feinheit aufgelegt werden;
   - Verbinden der Basisschicht (1) und der Oberschicht (2) durch Vernagelung oder durch Aufschmelzen, so dass das elastische Textil (9) in seiner Maschinenquerrichtung (MCD) Abschnitte mit unterschiedlicher Dehnbarkeit und/oder Elastizitätsmodul aufweist.

## Patentansprüche

1. Medizinisches Bekleidungsstück (10) zum Ausüben eines Kompressionsdrucks auf eine untere Extremität, mit
- einem einteiligen Zuschnitt (11) eines in Maschinenrichtung (MD) elastischen Textils (9) mit einem ersten und einem zweiten Abschnitt (K1, K2) in Maschinenquerrichtung (MCD), wobei der erste Abschnitt (K1) mit einer ersten Elastizität zum Anlegen an das Bein vorgesehen ist und der zweite Abschnitt (K2) mit einer zweiten Elastizität zum Anlegen an einen Fuß vorgesehen ist;
- Befestigungsmitteln (20), die dazu angepasst sind, durch überlappende Verbindung von entgegengesetzten Enden des Zuschnitts (11) in Maschinenrichtung (MD) das Bekleidungsstück (10) mittels Verschlusselementen (20a, 20b, 20c, 20d) die untere Extremität umschließend lösbar zu verschließen, **dadurch gekennzeichnet, dass**
das Bekleidungsstück (10) dazu angepasst ist, einen ersten Kompressionsdruck auf das Bein und einen zweiten Kompressionsdruck auf den Fuß auszuüben, wobei der erste Kompressionsdruck höher als der zweite Kompressionsdruck ist.

2. Medizinisches Bekleidungsstück (10) nach Anspruch 1, **dadurch gekennzeichnet, dass** der Zuschnitt (11) aus einem elastischen Textil (9) gefertigt ist, das einen Verbund (8) aus einer Basisschicht (1) und einer Oberschicht (2) aufweist, wobei die beiden Schichten (1,2) im ungedehnten Zustand mittels Nähwirkverfahren über in Maschinenrichtung (MD) laufende und in Maschinenquerrichtung (MCD) jeweils voneinander beabstandete elastische Nähfäden (3) miteinander verbunden sind, und wobei der Verbund (8) in Maschinenquerrichtung (MCD) des Nähwirkverfahrens in mindestens zwei unterscheidbare Abschnitte (K1, K2) unterteilt ist, wobei der erste Abschnitt (K1) und der zweite Abschnitt (K2) unterschiedliche Dehnbarkeiten in Maschinenrichtung (MD) aufweisen.

3. Medizinisches Bekleidungsstück (10) nach Anspruch 1 oder 2, **dadurch gekennzeichnet, dass** der Zuschnitt (11) einen dritten Abschnitt (K3) zum Anlegen an die Ferse aufweist, der eine dritte Elastizität aufweist.

4. Medizinisches Bekleidungsstück (10) nach einem der vorstehenden Ansprüche, **dadurch gekennzeichnet, dass** der erste und zweite Abschnitt (K1, K2) erste bzw. zweite Befestigungsmittel (20) aufweisen, so dass das elastische Textil (9) um das Bein bzw. den Fuß gelegt und auf sich selbst befestigt werden kann.

5. Medizinisches Bekleidungsstück (10) nach einem der vorstehenden Ansprüche, **dadurch gekennzeichnet, dass** der dritte Abschnitt (K3) keine Elastizität in Maschinenrichtung (MD) aufweist und dazu vorgesehen ist, einen Brückenabschnitt (16) zwischen erstem (12) und zweitem (14) Abschnitt auszubilden, um das Anlegen des Bekleidungsstückes (10) an Fuß und Bein zu erleichtern.

6. Medizinisches Bekleidungsstück (10) nach einem der vorstehenden Ansprüche, **dadurch gekennzeichnet, dass** der zweite Abschnitt (K2) ein Elastizitätsmodul von ≤ 6 kPa, bei 40% Dehnung und bei einer spezifischen Spannung von 1 N/cm aufweist.

7. Medizinisches Bekleidungsstück (10) nach einem der vorstehenden Ansprüche, **dadurch gekennzeichnet, dass** der erste Abschnitt (K1) für einen Therapiedruck von 2-8 kPa mit 40-45% ausgelegt ist.

8. Medizinisches Bekleidungsstück (10) nach einem der vorstehenden Ansprüche, **dadurch gekennzeichnet, dass** der erste Abschnitt (K1) ein Elastizitätsmodul von ≤ 40 kPa bei einer maximalen Spannung von 8 N/cm aufweist

9. Medizinisches Bekleidungsstück (10) nach einem der vorstehenden Ansprüche, **dadurch gekennzeichnet, dass** im ersten und zweiten Abschnitt (K1, K2) jeweils nur ein Befestigungsmittel (20) vorgesehen ist.

10. Medizinisches Bekleidungsstück (10) nach einem der vorstehenden Ansprüche, **dadurch gekennzeichnet, dass** das Befestigungsmittel (20) Verschlusselemente mit klebender Beschichtung, Klettverschlüsse oder Reißverschlüsse umfasst.

11. Medizinisches Bekleidungsstück (10) nach einem der vorstehenden Ansprüche, **dadurch gekennzeichnet, dass** der erste und/oder der zweite Abschnitt (K1, K2) Indikatoren oder Markierungen für die Verschlusselemente (20) aufweisen, um einen definierten Kompressionsdruck bei Anlegen des Bekleidungsstücks (10) auf Bein und Fuß auszuüben.

12. Medizinisches Bekleidungsstück (10) nach einem der vorstehenden Ansprüche, **dadurch gekennzeichnet, dass** der erste Abschnitt (K1) des Zuschnitts (11) eine rechteckige Form aufweist.

13. Medizinisches Bekleidungsstück (10) nach einem der vorstehenden Ansprüche, **dadurch gekennzeichnet, dass** der zweite Abschnitt (K2) des Zuschnitts (11) eine "American Football"-Form oder eine Zitronen-Form aufweist.

14. Medizinisches Bekleidungsstück (10) nach einem der vorstehenden Ansprüche, **dadurch gekennzeichnet, dass** der dritte Abschnitt (K3) des Zuschnitts (11) als Brückenabschnitt (16) zwischen erstem (12) und zweitem (14) Abschnitt sanduhrförmig ausgebildet ist, um eine Zugkraft von dem ersten Abschnitt (12) zu dem zweiten Abschnitt (14) breitflächig zu übertragen.

15. Verfahren zum Anlegen eines medizinischen Bekleidungsstücks (10) gemäß einem der vorstehenden Ansprüche, mit den Schritten:
- Auflegen des Zuschnitts (11) des medizinischen Bekleidungsstücks (10) auf einen flachen Untergrund;
- Betreten und Fixieren des zweiten Abschnitts (K2) durch einen Benutzer,
- Verschließen des Bekleidungsstücks (10) im Fußbereich durch den Benutzer mit einem definierten zweiten Kompressionsdruck;
- Greifen des proximalen Endes im ersten Abschnitt (K1) durch einen Benutzer, während er noch auf dem zweiten Abschnitt (K2) steht;
- Ziehen des ersten Abschnitts (K1) nach oben in Richtung des Rumpfes des Benutzers, wobei aufgrund der Einteiligkeit des Zuschnitts (11) ein Kraftschluss zwischen erstem und zweitem Abschnitt (K1, K2) zustande kommt, sodass sich der erste Abschnitt (K1) in Beinrichtung und Maschinenquerrichtung (MCD) des Zuschnitts (11) lokal definiert das Bein umschließt;
- Verschließen des Bekleidungsstücks (10) im Beinbereich durch den Benutzer mit einem definierten ersten Kompressionsdruck.

16. Verfahren zum Anlegen eines medizinischen Bekleidungsstücks nach Anspruch 15, **dadurch gekennzeichnet, dass** sich der dritte Abschnitt (K3), der an der Achillessehne der Ferse, Calcaneus, geführt wird, sich bei der Anwendung des medizinischen Bekleidungsstücks (10) nicht dehnt.

17. Verwendung des medizinisches Bekleidungsstück (10) nach einem der Ansprüche 1 bis 14 bei phlebo-lymphatischer Insuffizienz.
